(19) Europäisches Patentamt
European Patent Office
Office européen des brevets

(11) Publication number: **0 237 495**
**B1**

(12) **EUROPEAN PATENT SPECIFICATION**

(45) Date of publication of patent specification: **26.09.90**

(51) Int. Cl.$^5$: **C 07 C 219/22,**
C 07 D 209/28,
A 61 K 31/215, A 61 K 31/40

(21) Application number: **87830068.0**

(22) Date of filing: **27.02.87**

(54) **Halogenides of the ester of 2,N,N,N-dimethyl-alkyl-amino-ethanol with substituted acetic acid and pharmaceutical compositions having antiinflammatory and antiseptic activities containing same.**

(30) Priority: **04.03.86 IT 4771186**

(43) Date of publication of application:
**16.09.87 Bulletin 87/38**

(45) Publication of the grant of the patent:
**26.09.90 Bulletin 90/39**

(84) Designated Contracting States:
**AT BE CH DE ES FR GB GR LI LU NL SE**

(56) References cited:
**DE-A-3 023 206**
**US-A-4 150 137**

(73) Proprietor: **Sigma-Tau Industrie Farmaceutiche Riunite S.p.A.**
**47, Viale Shakespeare**
**I-00144 Rome (IT)**

(72) Inventor: **Reiner, Alberto**
**23/B Via Mentana**
**I-22100 Como (IT)**

(74) Representative: **Fassi, Aldo, Dr.**
**c/o Sigma-Tau Industrie Farmaceutiche Riunite S.p.A. Via Pontina, Km. 30, 400**
**I-00040 Pomezia (Roma) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 237 495 B1

## Description

This invention relates to halogenides of the ester of 2-N,N,N-dimethyl-alkyl-aminoethanol with substituted acetic acid of the general formula

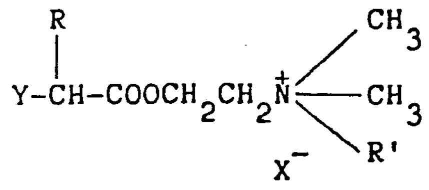

(I)

wherein:

R is hydrogen or $C_1$—$C_3$ lower alkyl,

R' is straight or branched $C_8$—$C_{16}$ alkyl,

$X^-$ is $Cl^-$, $Br^-$ or $I^-$

Y is a radical selected from p-isobutylphenyl, 3-benzoylphenyl, 1-(p-chlorobenzoyl)-2-methyl-5-methoxy-3-indolyl, 6-methoxy-2-naphthyl and 2-fluoro-4-biphenyl,

having high antiinflammatory and antiseptic properties.

This invention also relates to pharmaceutical compositions having antiinflammatory and antiseptic properties, which contain, as the active principle, a therapeutically effective amount of the compounds of formula (I) and a pharmacologically acceptable excipient. These antiinflammatory and antiseptic compositions are only intended for topical administration. Therefore, in addition to containing a compound of formula (I), as their active principle, they also comprise appropriate excipients that are suitable for the formation of topical compositions.

From DE—A—3 023 206 are known indometacine-dialkylamino-ethylesters which are useful as antiinflammatory and antirheumatic agents. Also from US—A—4 150 137 are known pyridylalkyl esters of 2-(p-isobutylphenyl) acetic or propionic acids useful as analgesics, antipyretics and antiinflammatory agents.

Because of their powerful antiinflammatory and antiseptic properties the compounds of formula (I) can be used particularly for the treatment of infectious diseases in which in addition to reducing the inflammatory process, it is desirable to prevent or abolish the growth of pathogenic microorganisms.

This kind of pathologies is typically represented by certain affections of the skin and the mucosae, such as:

distrophycally developing cutaneous ulcers (trophic, post-thrombotic, varicose, decubitus ulcers, etc. . . .)

burns

vaginitis in the presence or absence of microbial component

inflammation of soft tissues in the oral cavity and the upper respiratory tract, in the presence or absence of microbic component (stomatitis, tonsillitis, pharyngitis, etc. . . .).

Furthermore, the compounds of formula (I) can be used in the topical treatment of painful affections of the musculoskeletal system (traumas, osteoarthrosis, etc. . . .) due to their good anti-oedema and analgesic effects.

Preferred halogenides of the formula (I) are those wherein R is hydrogen or methyl, R' is octyl, decyl, dodecyl or cetyl, and $X^-$ is $Br^-$ or $I^-$.

Particularly preferred among these are:

the bromide of the ester of 2-N,N,N-dimethyl-octyl-amino-ethanol with p-isobutylphenyl-alpha-methylacetic acid of the formula

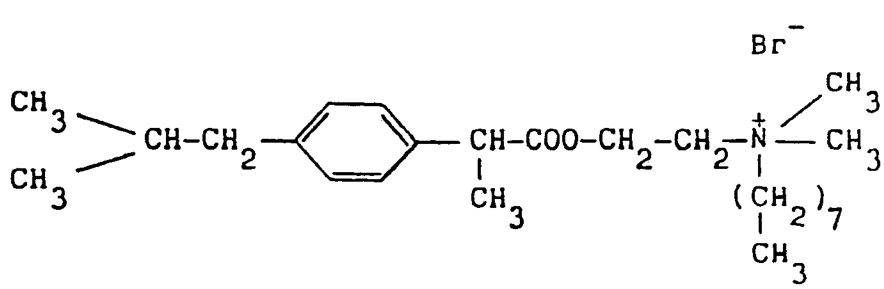

(hereinafter referred to as AF 150);

the iodide of the ester of 2-N,N,N-dimethyl-decyl-amino-ethanol with 1-(p-chlorobenzoyl)-2-methyl-5-methoxy-3-indolyl-acetic acid, of formula

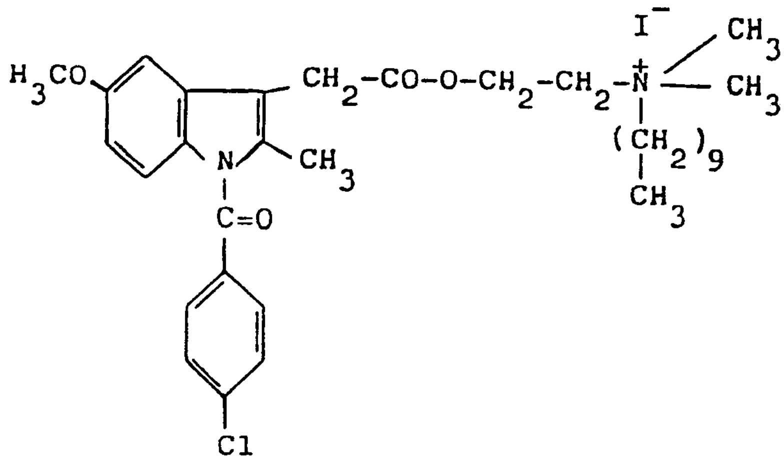

(hereinafter referred to as RE/8): and

the iodide of the ester of 2-N,N,N-dimethyl-dodecyl, amino-ethanol with 1-(p-chlorobenzoyl)-2-methyl-5-methoxy-3-indolyl-acetic acid, of formula

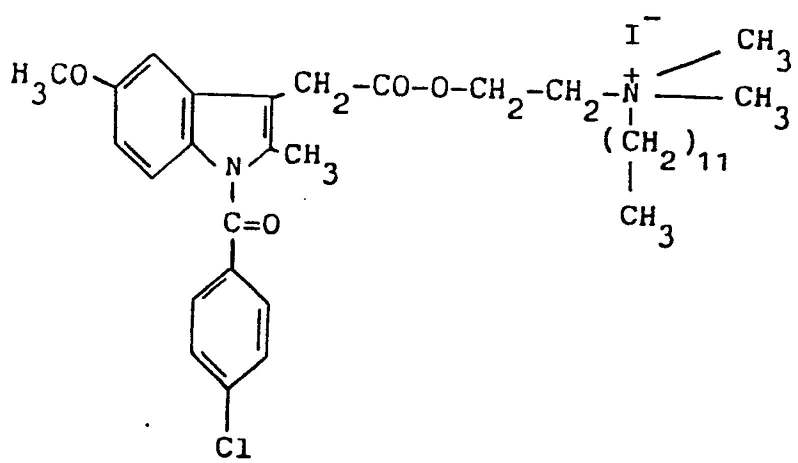

(referred to as RE/9 hereinafter).

The compounds of formula (I) are prepared by

(a) esterifying dimethyl-amino-ethanol with the acid chloride of an acid having the general formula

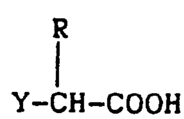

(II)

in which R and Y are as defined above, and

(b) reacting the thus obtained ester with an alkyl halogenide of the general formula

$$R'X \qquad (III)$$

in which R' and X are as defined above.

Non limiting examples of acids of formula (II) are alpha-methyl-4-(2-methyl propyl) benzenacetic, 2-(3-benzoylphenyl) propionic, 1-(p-chlorobenzoyl)-5-methoxy-2-methyl-2-indolyl-acetic, (+)-6-methoxy-alpha-methyl-2-naphthalenacetic and 2-fluoro-alphamethyl-4-biphenylacetic acid.

More particularly, a stoichiometric amount of dimethylamino-ethanol dissolved in $CHCl_3$ is added dropwise to a solution of the acid chloride of the acid (II) in $CHCl_3$. The resulting reaction mixture should be maintained at a temperature in the range of from 20° to 60°C. Then, the mixture is kept under stirring at 40—50°C for 2—3 hours. The mixture is cooled and the base is freed with a stoichiometric amount of soda dissolved in as great a water volume as half volume of the reaction mixture. After vigorous stirring for

30—60 min, the organic phase is separated and dried on $Na_2SO_4$ (the pH of the aqueous phase should be adjusted to 8 in order to purify the ester from any unreacted acid). The organic phase is concentrated to dryness and the residue taken up with ethyl acetate, whereupon twice the stoichiometric amount of the alkyl halogenide (III) is added thereto. The mixture is refluxed for 18—24 hours, then allowed to cool. The liquid phase is filtered off, and the compound (1) washed with ethyl acetate.

The following non-limiting examples illustrate the preparation of some compounds of formula (II).

Example 1

Preparation of compound AF 150

A suspension of the acid chloride of alpha-methyl-4-(2-methyl propyl) benzenacetic acid (50 g; 0.218 moles) in 50 ml of $CHCl_3$ was prepared.

A solution of dimethylaminoethanol (19.6 g; 0.218 moles) in 20 ml of $CHCl_3$ was added to the suspension over an hour and a half period. The end temperature of the reaction mixture was 42°C. The reaction mixture was kept under stirring for 2 hours at 45°C, whereupon it was allowed to cool and, then, the stoichiometric quantity of NaOH dissolved in 60 ml water was added thereto; the resulting heterogeneous mixture was cold stirred for 30 min, the organic phase separated, dried on $Na_2SO_4$ and concentrated to dryness, thus giving the ester as a chromatographically pure oil (45 g, 73% yield).

Eluent: $CHCl_3/MeOH/NH_3$ 40:9:1

Plates: Merck 5554

Octyl bromide, 43 grams, was added to a solution of 31 grams of the ester dissolved in ethyl acetate and the resulting reaction mixture refluxed for 10 hours. The resulting clear mixture was taken up with 100 ml ethyl acetate, heated to its boiling temperature and then allowed to cool. Upon filtration, 26 grams (49% yield) of the title compound were obtained as a white crystalline compound. Melting point: 104—109°C.

Example 2

Preparation of the compound RE/8

The procedure of Example 1 was followed except that 28.7 g (0.076 moles) of the acid chloride of 1-(p-chlorobenzoyl)-5-methoxy-2-methyl-indole-3-acetic acid was used for preparing the ester. The ester was reacted with decyl iodide to obtain the title compound as a creamwhite crystalline product (yield: 57%). Melting point: 187—194°C.

Example 3

Preparation of the compound RE/9

The same procedure as that in Example 2 was followed except that the ester was reacted with dodecyl iodide. The title compound was obtained (60% yield) as a light yellow crystalline product. Melting point: about 160°C.

Pharmacological Tests

There are reported hereinbelow the results of pharmacological tests on some of the compounds of formula (I) which show microbiological activity comparable to that of the most widely used antiseptics, associated to significant antiinflammatory effect.

In the pharmcological tests, both female and male Swiss mice (25—30 g) and Wistar rats (140—170 g) were used. Since no significant differences in response between the male and female animal groups was noted, the test results were pooled. Chlorexidine gluconate and Ibuprofen (acid) were used as reference products.

All of the doses and concentrations are referred to the salts.

When administered via the intraperitoneal and oral routes, 10 ml/kg of 0.5% suspensions in methylcellulose were administered. When administered via the intravenous route, the products were dissolved in saline solution (0.9% NaCl). The variance analysis and the Student's t-test were used for statistical processing of the results.

The following tests were carried out:

(1) Topical tolerability

The test was carried out on rats using two different test procedures. The products were tested at 1% concentration. According to the first procedure, 1 ml of the test suspensions were injected subcutaneously (s.c.) in the side of animals. 4 or 24 hours following administration, the animals were sacrificed, and the degree of subcutaneous irritation estimated.

The presence of erythema, oedema and necrosis was assessed based on the following arbitrary rating scale

0: no response
1: moderate response
2: sharp response
3: marked response
4: extreme response

According to the second test procedure, 1 ml of the suspensions were administered intra-peritoneally at the same time, 1 ml of 0.1% Blue Evans was administered i.v. to the animals. 4 hours following

administration the rats were sacrificed and the peritoneum washed with 10 ml of saline solution. The washing liquid was measured, then centrifuged at 5,000 rpm for 10 min. The volume, optical density (O.D.) at 640 nm and irritation coefficient (volume × O.D. at 640 nm) of each sample were noted.

(2) Antiexudative activity

The test was carried out in the rat. The products were orally administered 1 hour before the intraperitoneal injection of 0.5 ml of 1% acetic acid. Along with the irritant agent, 1 ml of 0.1% Blue Evans was injected i.v. to the rats. The same procedure as that described above was followed for collecting the peritoneal exudate and rating the irritation degree.

(3) Acute toxicity

The test was carried out in the mouse. The products were administered by intraperitoneal injection. The death rate was assessed daily over a period of 14 days following treatment.

## Results

(1.1) Topical tolerability

The results as to tolerability in the rat subcutaneous tissues are reported in Table 1. The results of tolerability in rat peritoneum are shown in Table 2. The data demonstrate that in rat subcutaneous tissues the compounds of the invention cause a light to moderate degree of irritation, that is, of the same order of magnitude as that observed with methylcellulose. The data relating to rat peritoneum are in substantial agreement with those relating to the rat subcutaneous tissues.

It is to be noted that Chlorexidine causes an intense exudative reaction to occur in the rat peritoneum, insofar as it brings about an increase in response which is 442% higher than that recorded for the control group.

(2.1) Antiexudative activity

The results obtained for acetic acid-induced peritoneal exudation are illustrated in Table 3. Ibuprofen was used as reference product. AF 150 is the most active derivative in this test. In fact, 400 mg/kg orally and 200 mg/kg orally inhibit the inflammatory reaction to a statistically significant extent, 51% and 37% respectively. RE 8 and RE 9 derivatives exhibit a statistically significant effect even though their activity is less potent than that of AF 150. In comparative tests, Ibuprofen already exhibits its most potent effect at 200 mg/kg orally; in fact its percent inhibition does not increase even though the dosage exceeds this threshold. A study of the antiinflammatory activity clearly showed that AF 150, RE 8 and RE 9, when administered orally, inhibit acetic acid-induced peritonitis. In comparative tests, Ibuprofen proved more active than the derivatives under examination including AF 150 whose antiinflammatory moiety of the molecule is Ibuprofen.

However, it has to be kept in mind that, in the products under examination, the portion pertaining to the antiinflammatory moiety is about 50% of the whole structure. Consequently, with regard to the antiexudative activity, the doses of the compounds according to the invention, the number of mg/kg being the same in either case, are half the doses of Ibuprofen.

(3.1) Acute toxicity

Acute toxicity data are illustrated in Table 4. The tests were run in mouse, the compounds being administered via the intraperitoneal route.

Acute toxicity of the molecules under examination is relatively high, in fact, doses of 30—60 mg/kg i.p. bring about death of 80—100% of animals.

In particular, AF 150 and RE 8 do not bring about death at 15.6 mg/kg i.p. whereas at 31.2 mg/kg i.p. they are lethal to 100% of animals. RE 9 is comparatively less toxic: at 31.2 mg/kg i.p., it does not cause death whereas at 62.5 mg/kg i.p. it is lethal to 83.3% of animals.

However, it should be noted that the pharmaceutical compositions having antiinflammatory and antiseptic activities according to the present invention which contain one of the compounds of formula (I) as their active principle, are only intended for topical administration. Consequently, the acute toxicity data are only significant in relation to the amounts of compound that will actually be adsorbed systemically following topical treatment.

It has been found that product plasma levels that are detected following topical treatment are far below those observed following systemic treatment, and are generally less than a tenth of the plasma level attained via systemic administration.

TABLE 1

Topical tolerability in rat subcutaneous tissue (Reference compond: Methylcellulose)

| Compound | Conc. % | Rats n. | Irritation 4h | 24h |
|---|---|---|---|---|
| Methylcellulose | / | 10 | 2 | 1 |
| AF 150 | 1 | 10 | 2 | 1 |
| RE 8 | 1 | 10 | 2 | 2 |
| RE 9 | 1 | 10 | 2 | 2 |

TABLE 2

Topical tolerability in rat peritoneum (Reference compound: Chlorexidine)

| Compound | Conc. % | Rats n. | Percent increase in response with respect to controls |
|---|---|---|---|
| Chlorexidine | 1 | 10 | 441 |
| AF 150 | 1 | 10 | 185 |
| RE 8 | 1 | 10 | 97 |
| RE 9 | 1 | 10 | 95 |

TABLE 3

Antiexudative activity in rat (Reference product: Ibuprofen)

| Compound | Dosage mg/kg orally | Rats N. | % Inhibition with respect to controls | P |
|---|---|---|---|---|
| ibuprofen | 400 | 10 | 58 | >0.001[1)] |
| | 200 | 10 | 60 | >0.01[2)] |
| AF 150 | 400 | 9 | 51 | >0.01[1)] |
| | 200 | 10 | 37 | >0.01[2)] |
| RE 8 | 400 | 8 | 46 | >0.02[1)] |
| | 200 | 10 | 31 | >0.05[2)] |
| RE 9 | 400 | 7 | 40 | >0.05[1)] |
| | 200 | 10 | 35 | >0.05[2)] |

[1)] Student's t-test
[2)] Duncan test

TABLE 4
Acute toxicity in mouse

| Product | Dosae mg/kg i.p. | Mice N. | Mortality | |
|---|---|---|---|---|
| AF 150 | 125 | 6 | 5/6 | 83.3% |
| | 62.5 | 6 | 6/6 | 100 % |
| | 31.2 | 6 | 6/6 | 100 % |
| | 15.6 | 6 | 0/6 | 0 % |
| | 7.8 | 6 | 0/6 | 0 % |
| RE 8 | 250 | 6 | 6/6 | 100 % |
| | 125 | 6 | 5/6 | 83.3% |
| | 62.5 | 6 | 6/6 | 100 % |
| | 31.2 | 6 | 6/6 | 100 % |
| | 15.6 | 6 | 0/6 | 0 % |
| | 7.8 | 6 | 0/6 | 0 % |
| RE 9 | 500 | 6 | 6/6 | 100 % |
| | 250 | 6 | 5/6 | 83.3% |
| | 125 | 6 | 6/6 | 100 % |
| | 62.5 | 6 | 5/6 | 83.3% |
| | 31.2 | 6 | 0/6 | 0 % |

i.p. — intraperitoneal

Microbiological Tests

The activity *in vitro* has been evaluated on the following microorganisms:

*Gram-Positive Bacteria:*
Staphylococcus aureus
Staphylococcus epidermidis
Streptococcus faecalis
Streptococcus pyogenes
Bacillus substilis
Bacillus cereus mycoides
Sarcina lutea
Bacillus antracis

*Gram-Negative Bacteria:*
Escherichia coli
Salmonella typhimurium
Salmonella cholerae suis
Klebsiella pneumoniae
Proteus vulgaris
Pseudomonas aeruginosa
Bordetella bronchiseptica
Pseudomonas maltuphylia

*Blastomycetes:*
Candida albicans
Candida utilis
Rhodotorula rubra
Cryptococcus neoformans
Saccharomyces cerevisiae

*Ifomycetes:*
Aspergillus niger
Penicillium funicolosum

Chlorexidine gluconate was used as reference compound.

Compounds were tested at 5, 10, 20, 40 and 80 µg/ml. Results are shown in Tables 5, 6 and 7.

As it can be seen, compounds AF 150 and RE 9 are the most active compounds, because more potent than Chlorexidine against Gram-positive bacteria, Blastomycetes and Ifomycetes, and as potent as Chlorexidine against Gram-negative bacteria.

Compound RE 8 is more potent than Chlorexidine against Gram-positive bacteria, Blastomycetes and Ifomycetes, and less potent than Chlorexidine on Gram-positive bacteria.

TABLE 5

Determination of Minimum Inhibiting Concentration

| Strain | Repository numbers | Sample AF 150 | Reference compound Chlorexidine Gluconate |
|---|---|---|---|
| Gram Positive Bacteria | | | |
| Staphylococcus aureus | ATCC 6538P | ≤5.0 | ≤5.0 |
| Staphylococcus epidermidis | BB 0223 | 10.0 | 10.0 |
| Streptococcus faecalis | ATCC 8043 | 20.0 | 20.0 |
| Streptococcus pyogenes | ATCC 12380 | 20.0 | 10.0 |
| Bacillus subtilis | ATCC 6633 | 10.0 | ≤5.0 |
| Bacillus cereus mycoides | ISM 65/42 | 10.0 | 10.0 |
| Sarcina lutea | ATCC 9341 | ≤5.0 | 20.0 |
| Bacillus antracis | ISM 68/35 | 10.0 | 20.0 |
| Gram Negative Bacteria | | | |
| Escherichia coli | K 12 | 80.0 | 10.0 |
| Salmonella typhimurium | ATCC 15277 | 80.0 | 20.0 |
| Salmonella cholerae suis | ATCC 10708 | 80.0 | 10.0 |
| Klebsiella pneumoniae | ATCC 10031 | 10.0 | 40.0 |
| Proteus vulgaris | ATCC 6897 | 10.0 | 40.0 |
| Pseudomonas aeruginosa | ATCC 14502 | 10.0 | 80.0 |
| Bordetella bronchiseptica | ATCC 4617 | 10.0 | 40.0 |
| Pseudomonas maltophilia | ATCC 13637 | 40.0 | 80.0 |
| Blastomycetes | | | |
| Candida albicans | ATCC 10231 | ≤5.0 | 20.0 |
| Candida utilis | BB 0173 | ≤5.0 | 40.0 |
| Rhodotorula rubra | ISM 7198 | ≤5.0 | 40.0 |
| Cryptococcus neoformans | ISM 7182 | ≤5.0 | 40.0 |
| Saccharomyces cerevisiae | BB 0193 | ≤5.0 | 80.0 |
| Ifomycetes | | | |
| Aspergillus niger | ATCC 9642 | 10.0 | 80.0 |
| Penicillium funicolosum | ATCC 9644 | 10.0 | >80.0 |

TABLE 6

Determination of Minimum Inhibiting Concentration

| Strain | Repository numbers | Sample RE 9 | Reference compound Chlorexidine Gluconate |
|---|---|---|---|
| Gram Positive Bacteria | | | |
| Staphylococcus aureus | ATCC 6538P | ≤5.0 | ≤5.0 |
| Staphylococcus epidermidis | BB 0223 | ≤5.0 | 10.0 |
| Streptococcus faecalis | ATCC 8043 | ≤5.0 | 20.0 |
| Streptococcus pyogenes | ATCC 12380 | ≤5.0 | 10.0 |
| Bacillus subtilis | ATCC 6633 | ≤5.0 | ≤5.0 |
| Bacillus cereus mycoides | ISM 65/42 | ≤5.0 | 10.0 |
| Sarcina lutea | ATCC 9341 | ≤5.0 | 20.0 |
| Bacillus antracis | ISM 68/35 | ≤5.0 | 20.0 |
| Gram Negative Bacteria | | | |
| Escherichia coli | K 12 | 80.0 | 10.0 |
| Salmonella typhimurium | ATCC 15277 | 20.0 | 20.0 |
| Salmonella cholerae suis | ATCC 10708 | 20.0 | 10.0 |
| Klebsiella pneumoniae | ATCC 10031 | 80.0 | 40.0 |
| Proteus vulgaris | ATCC 6897 | 20.0 | 40.0 |
| Pseudomonas aeruginosa | ATCC 14502 | 80.0 | 80.0 |
| Bordetella bronchiseptica | ATCC 4617 | 80.0 | 40.0 |
| Pseudomonas maltophilia | ATCC 13637 | 80.0 | 80.0 |
| Blastomycetes | | | |
| Candida albicans | ATCC 10231 | ≤5.0 | 20.0 |
| Candida utilis | BB 0173 | ≤5.0 | 40.0 |
| Rhodotorula rubra | ISM 7198 | ≤5.0 | 40.0 |
| Cryptococcus neoformans | ISM 7182 | ≤5.0 | 40.0 |
| Saccharomyces cerevisiae | BB 0193 | ≤5.0 | 80.0 |
| Ifomycetes | | | |
| Aspergillus niger | ATCC 9642 | ≤5.0 | 80.0 |
| Penicillium funicolosum | ATCC 9644 | ≤5.0 | >80.0 |

TABLE 7

Determination of Minimum Inhibiting Concentration

| Strain | Repository numbers | Sample RE 8 | Reference compound Chlorexidine Gluconate |
|---|---|---|---|
| Gram Positive Bacteria | | | |
| Staphylococcus aureus | ATCC 6538P | ≤5.0 | ≤5.0 |
| Staphylococcus epidermidis | BB 0223 | ≤5.0 | 10.0 |
| Streptococcus faecalis | ATCC 8043 | 10.0 | 20.0 |
| Streptococcus pyogenes | ATCC 12380 | 10.0 | 10.0 |
| Bacillus subtilis | ATCC 6633 | 10.0 | ≤5.0 |
| Bacillus cereus mycoides | ISM 65/42 | 10.0 | 10.0 |
| Sarcina lutea | ATCC 9341 | 10.0 | 20.0 |
| Bacillus antracis | ISM 68/35 | 10.0 | 20.0 |
| Gram Negative Bacteria | | | |
| Escherichia coli | K 12 | >80.0 | 10.0 |
| Salmonella typhimurium | ATCC 15277 | 20.0 | 20.0 |
| Salmonella cholerae suis | ATCC 10708 | 20.0 | 10.0 |
| Klebsiella pneumoniae | ATCC 10031 | >80.0 | 40.0 |
| Proteus vulgaris | ATCC 6897 | 80.0 | 40.0 |
| Pseudomonas aeruginosa | ATCC 14502 | 80.0 | 80.0 |
| Bordetella bronchiseptica | ATCC 4617 | 80.0 | 40.0 |
| Pseudomonas maltophilia | ATCC 13637 | 80.0 | 80.0 |
| Blastomycetes | | | |
| Candida albicans | ATCC 10231 | ≤5.0 | 20.0 |
| Candida utilis | BB 0173 | ≤5.0 | 40.0 |
| Rhodotorula rubra | ISM 7198 | ≤5.0 | 40.0 |
| Cryptococcus neoformans | ISM 7182 | ≤5.0 | 40.0 |
| Saccharomyces cerevisiae | BB 0193 | ≤5.0 | 80.0 |
| Ifomycetes | | | |
| Aspergillus niger | ATCC 9642 | ≤5.0 | 80.0 |
| Penicillium funicolosum | ATCC 9644 | ≤5.0 | >80.0 |

The most suitable pharmaceutical preparations for topical administration of the compounds according to this invention, having regard to the therapeutic fields of application, are as follows:

| In gynaecology | In trauma-rheumatology |
|---|---|
| ready-to-use vaginal douche | gel |
| small bags | cream |
| concentrated solution | spray lotion |
| spray cream | |
| ovules | |

| In stomatology | In dermatology |
|---|---|
| collutory | ointment |
| | paste |
| | spray cream |

For burn treatment

- cream
- spray cream
- medicated gauze

Concentrations of products range from 0.5 to 1% by weight, depending on the particular pharmaceutical preparation. Choice of the most appropriate excipients depends on the specific

pharmaceutical preparation and will be obvious to one of ordinary skill in the pharmaceutical technology. As should be apparent excipients compatible with human use shall be utilized, preference being given to the excipients already listed in the Italian Pharmacopoeia or the Pharmacopoeias of main European and non-European countries.

**Claims for the Contracting States: BE CH DE FR GB LI LU NL SE**

1. Halogenides of the ester of 2-N,N,N-dimethyl-alkyl-amino-ethanol with substituted acetic acid, of the general formula

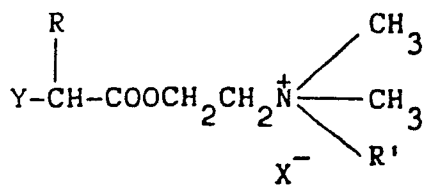

(I)

wherein
R is hydrogen or lower $C_1$—$C_3$ alkyl,
R′ is straight or branched $C_8$—$C_{16}$ alkyl,
$X^-$ is $Cl^-$, $Br^-$ or $I^-$,
Y is a radical selected from p-isobutylphenyl, 3-benzoyl-phenyl, 1-(p-chlorobenzoyl)-2-methyl-5-methoxy-3-indolyl, 6-methoxy-2-naphthyl and 2-fluoro-4-biphenyl.

2. A halogenide according to claim 1, wherein R is hydrogen or methyl, R′ is octyl, decyl, dodecyl or cetyl, and $X^-$ is $Br^-$ or $I^-$.

3. A halogenide of claim 2, the bromide of the ester of 2-N,N,N-dimethyl-octyl-aminoethanol with p-isobutylphenyl-alpha-methylacetic acid of formula

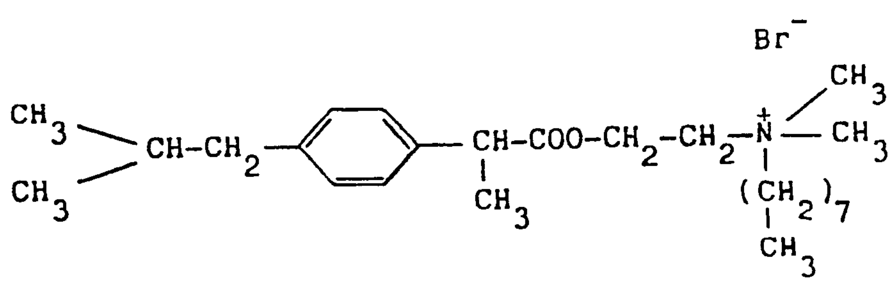

4. A halogenide of claim 2, the iodide of the ester of 2-N,N,N-dimethyl-decyl-aminoethanol with 1-(p-chlorobenzoyl)-2-methyl-5-methoxy-3-indolyl acetic acid, of the formula

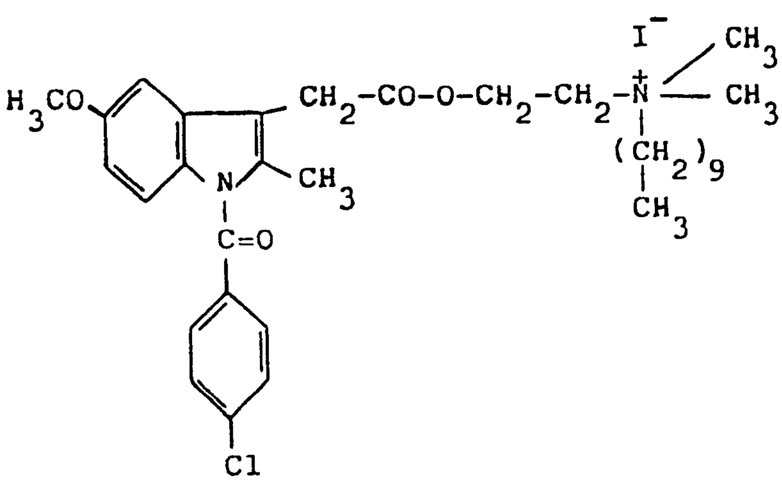

5. A halogenide according to claim 2, the iodide of the ester of 2-N,N,N-dimethyl-dodecyl-amino-ethanol with 1-(p-chlorobenzoyl)-2-methyl-5-methoxy-3-indolyl acetic acid, of the formula

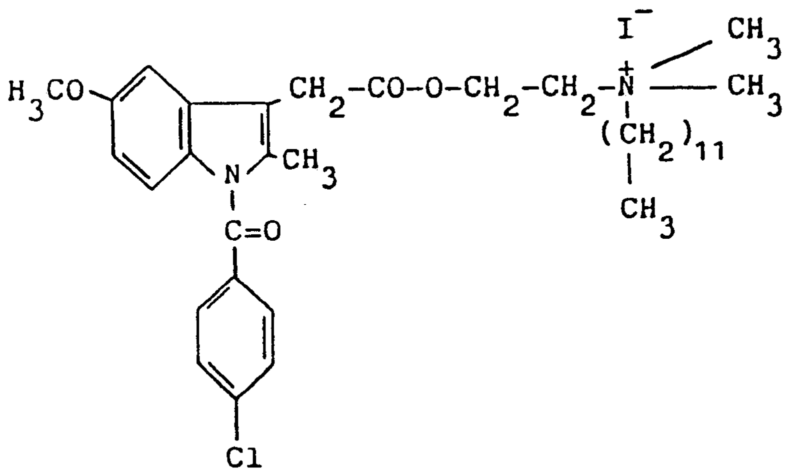

6. A pharmaceutical composition having antiinflammatory and antiseptic activities, intended for topical administration, which comprises

(a) a therapeutically effective amount of a halogenide of the ester of 2-N,N,N-dimethyl-alkyl-amino-ethanol with substituted acetic acid, of the general formula

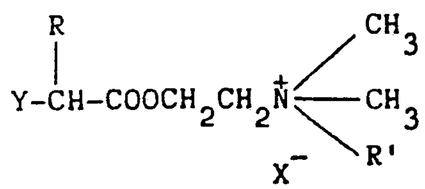

(I)

wherein

R is hydrogen or lower $C_1$—$C_3$ alkyl,

R' is straight or branched $C_8$—$C_{16}$ alkyl,

$X^-$ is $Cl^-$, $Br^-$ or $I^-$,

Y is a radical selected from p-isobutylphenyl, 3-benzoyl-phenyl, 1-(p-chlorobenzoyl)-2-methyl-5-methoxy-3-indolyl, 6-methoxy-2-naphthyl and 2-fluoro-4-biphenyl, and

(b) a pharmaceutically acceptable excipient suitable for preparing compositions for topical use.

**Claim for the Contracting States: AT ES GR**

A process for preparing halogenides of the ester of 2-N,N,N-dimethyl-alkyl-amino-ethanol with substituted acetic acid, of the general formula

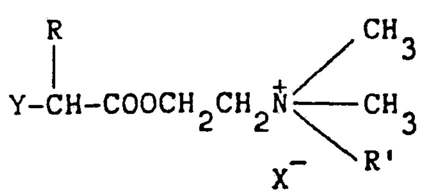

(I)

wherein

R is hydrogen or lower $C_1$—$C_3$ alkyl,

R' is straight or branched $C_8$—$C_{16}$ alkyl,

$X^-$ is $Cl^-$, $Br^-$ or $I^-$,

Y is a radical selected from p-isobutylphenyl, 3-benzoyl-phenyl, 1-(p-chlorobenzoyl)-2-methyl-5-methoxy-3-indolyl, 6-methoxy-2-naphthyl and 2-fluoro-4-biphenyl, characterized in that it comprises the steps of:

(a) esterifying dimethyl-amino-ethanol with the acid chloride of an acid of the general formula

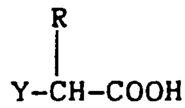

(II)

wherein R and Y are as defined above and

(b) reacting the thus obtained ester with an alkyl halide of general formula

R'X (III)

wherein R' and X are as defined hereinabove.

**Patentansprüche für die Vertragsstaaten: BE CH DE FR GB LI LU NL SE**

1. Halogenide des Esters von 2-N,N,N-Dimethyl-alkyl-aminoethanol mit substituierter Essigsäure der allgemeinen Formel

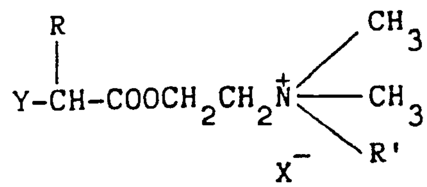

(I)

worin

R Wasserstoff oder niedriges $C_{1-3}$-Alkyl ist;

R' geradkettiges oder verzweigtes $C_{8-16}$-Alkyl ist;

$X^-$ $Cl^-$, $Br^-$ oder $I^-$ ist;

Y ein Rest ist, ausgewählt aus p-Isobutylphenyl, 3-Benzoylphenyl, 1-(p-Chlorbenzoyl)-2-methyl-5-methoxy-3-indolyl, 6-Methoxy-2-naphthyl und 2-Fluor-4-biphenyl.

2. Halogenid gemäss Anspruch 1, worin R Wasserstoff oder Methyl, R' Octyl, Decyl, Dodecyl oder Cetyl, und $X^-$ $Br^-$ oder $I^-$ ist.

3. Halogenid gemäss Anspruch 2, nämlich das Bromid des Esters von 2-N,N,N-Dimethyl-octyl-amino-ethanol mit p-Isobutylphenyl-alpha-methylessigsäure der Formel

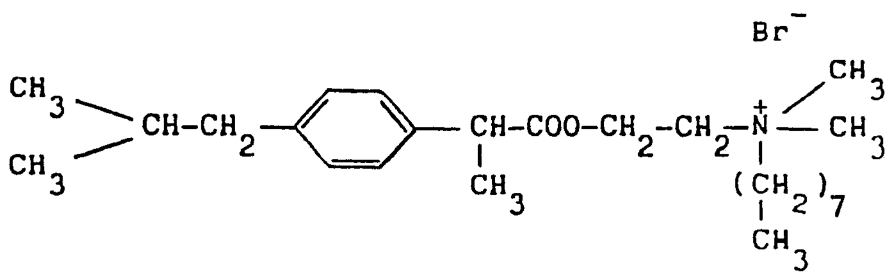

4. Halogenid gemäss Anspruch 2, nämlich das Jodid des Esters von 2-N,N,N-Dimethyl-decyl-amino-ethanol mit 1-(p-Chlorbenzoyl)-2-methyl-5-methoxy-3-indolylessigsäure der Formel

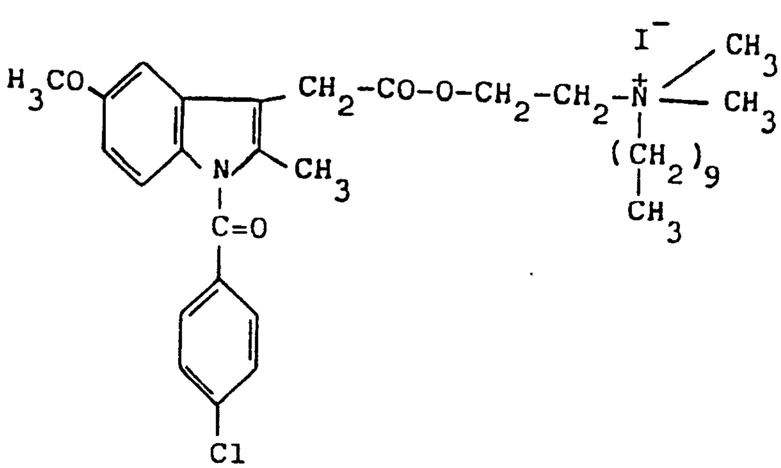

5. Halogenid gemäss Anspruch 2, nämlich das Jodid des Esters von 2-N,N,N-Dimethyl-dodecyl-aminoethanol mit 1-(p-Chlorbenzoyl)-2-methyl-5-methoxy-3-indolylessigsäure der Formel

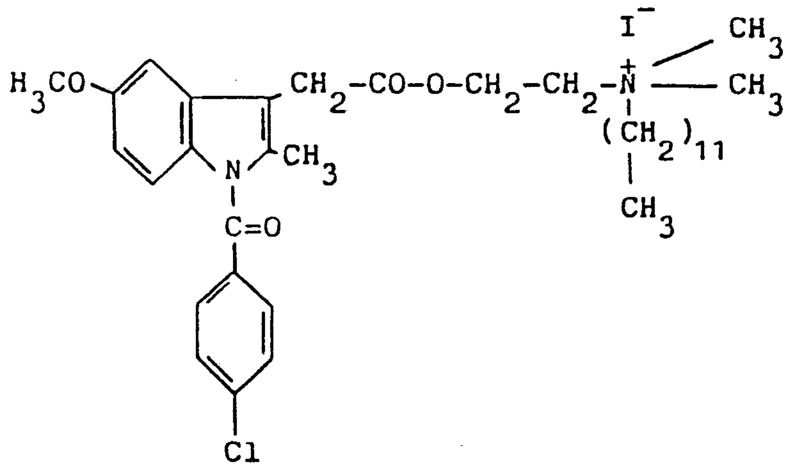

6. Pharmazeutische Zusammensetzung mit entzündungshemmender und antiseptischer Wirkung, bestimmt zur äusseren Anwendung, umfassend

(a) eine therapeutisch wirksame menge eines Halogenides des Esters von 2-N,N,N-Dimethylalkylaminoethanol mit substituierter Essigsäure der allgemeinen Formel (I)

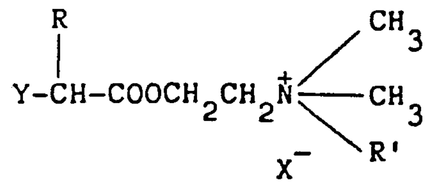

(I)

worin

R Wasserstoff oder niedriges $C_{1-3}$-Alkyl ist;

R' geradkettiges oder verzweigtes $C_{8-16}$-Alkyl ist;

$X^-$ $Cl^-$, $Br^-$ oder $I^-$ ist;

Y ein Radikal ist, ausgewählt aus p-Isobutylphenyl, 3-Benzoylphenyl, 1-(p-Chlorbenzoyl)-2-methyl-5-methoxy-3-indolyl, 6-Methoxy-2-naphthyl und 2-Fluor-4-biphenyl; und

(b) einen pharmazeutisch akzeptierbaren Exzipienten, der zur Herstellung von Zusammensetzungen für die topische Verwendung geeignet ist.

**Patentansprüche für die Vertragsstaaten: AT ES GR**

Verfahren zur Herstellung von Halogeniden des Esters von 2-N,N,N-Dimethylalkylaminoethanol mit substituierter Essigsäure der allgemeinen Formel (I)

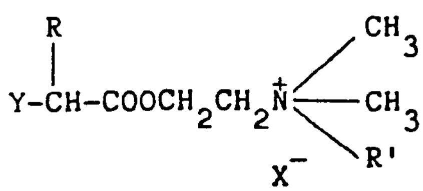

(I)

worin

R Wasserstoff oder niedriges $C_{1-3}$-Alkyl ist;

R' geradkettiges oder verzweigtes $C_{8-16}$-Alkyl ist;

$X^-$ $Cl^-$, $Br^-$ oder $I^-$ ist;

Y ein Rest ist, ausgewählt aus p-Isobutylphenyl, 3-Benzoylphenyl, 1-(p-Chlorbenzoyl)-2-methyl-5-methoxy-3-indolyl, 6-Methoxy-2-naphthyl und 2-Fluor-4-biphenyl, dadurch gekennzeichnet, dass es dei folgenden Schritte umfasst:

(a) Verestern von Dimethylaminoethanol mit dem Säurechlorid einer Säure der allgemeinen Formel (II)

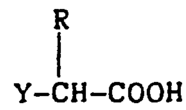

(II)

worin R und Y die vorerwähnten Bedeutungen haben, und

(b) Umsetzen des so erhaltenen Esters mit einem Alkylhalogenid der allgemeinen Formel

$$R'X \qquad (III)$$

worin R′ und X die vorerwähnten Bedeutungen haben.

**Revendications pour les Etats Contractants: BE CH DE FR GB LI LU NL SE**

1. Halogénures de l'ester de 2-N,N,N-diméthyl-alkyl-amino-éthanol avec de l'acide acétique substitué, de formule générale:

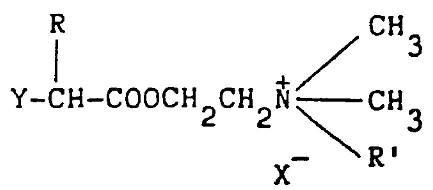

(I)

dans laquelle

R est l'hydrogéne ou un alcoyle inférieur en $C_1$—$C_3$,

R′ est un alcoyle linéaire ou ramifié en $C_8$—$C_{16}$,

$X^-$ est $Cl^-$, $Br^-$ or $I^-$,

Y est un radical choisi parmi p-isobutylphényl, 3-benzoyl-phényl, 1-(p-chlorobenzoyl)-2-méthyl-5-méthoxy-3-indolyl, 6-méthoxy-2-naphthyl et 2-fluoro-4-biphényl.

2. Halogénure selon la revendication 1, dans lequel R est un hydrogène ou un méthyl, R′ est octyl, décyl, dodécyl ou cétyl, et $X^-$ est $Br^-$ ou $I^-$.

3. Halogénure selon la revendication 2, qui est le bromure de l'ester de 2-N,N,N-diméthyl-octyl-amino-éthanol avec l'acide p-isobutylphényl-alpha-méthylacétique de formule:

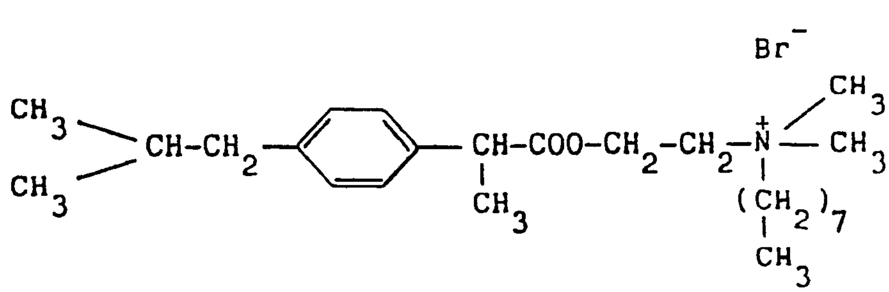

4. Halogénure selon la revendication 2, qui est l'iodure de l'ester de 2-N,N,N-diméthyl-décyl-amino-éthanol avec l'acide 1-(p-chlorobenzoyl)-2-méthyl-5-méthoxy-3-indolyl acétique de formule:

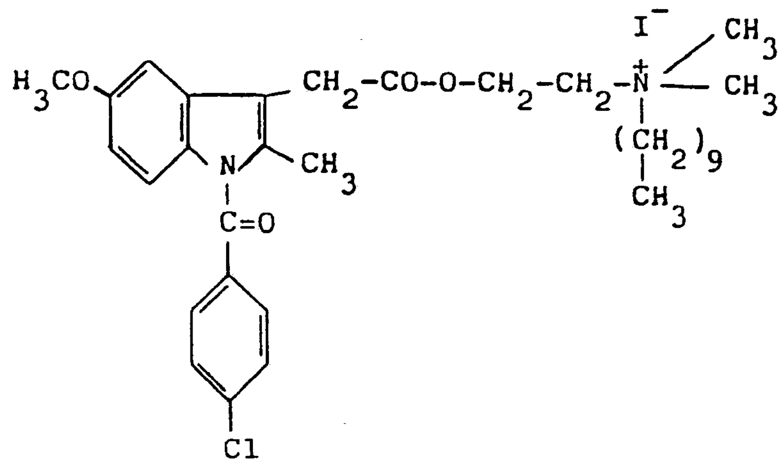

5. Halogénure selon la revendication 2, qui est l'iodure de l'ester de 2-N,N,N-diméthyl-dodécyl-amino-éthanol avec l'acide 1-(p-chlorobenzoyl)-2-méthyl-5-méthoxy-3-indolyl acétique de formule:

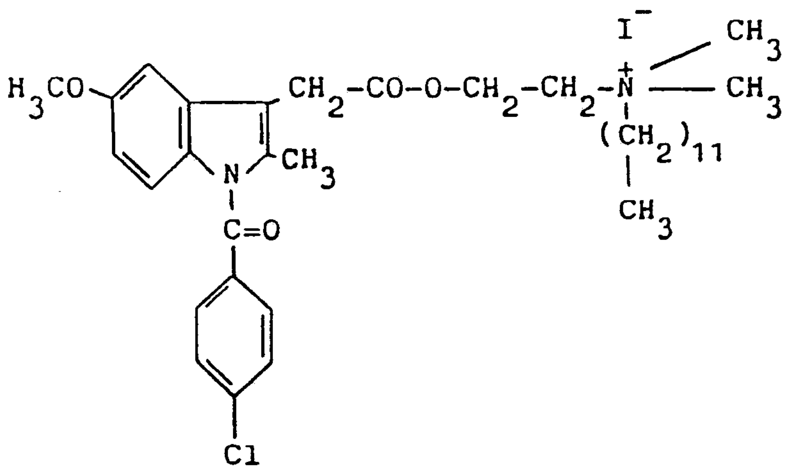

6. Composition pharmaceutique ayant des activités antiinflammatoires et antiseptiques, destinée à l'administration topique qui comprend:

(a) une quantité thérapeutiquement efficace d'un halogénure de l'ester 2-N,N,N-diméthyl-alkyl-amino-éthanol avec de l'acide acétique de formule générale:

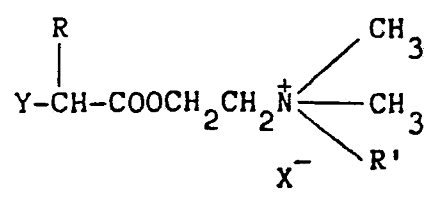

(I)

dans laquelle:

R est l'hydrogéne ou un alcoyle inférieur en $C_1$—$C_3$,

R' est un alcoyle linéaire ou ramifié en $C_8$—$C_{16}$,

$X^-$ est $Cl^-$, $Br^-$ or $I^-$,

Y est un radical choisi parmi p-isobutylphényl, 3-benzoyle-phényl, 1-(p-chlorobenzoyl)-2-méthyl-5-méthoxy-3-indolyl, 6-méthoxy-2-naphthyl et 2-fluoro-4-biphényl, et

(b) un excipient pharmaceutiquement acceptable approprié pour la préparation de compositions à usage topique.

**Revendications pour les Etats Contractants: AT ES GR**

Procédé de préparation d'halogénures de l'ester de 2-N,N,N-diméthyl-alkyl-amino-éthanol avec de l'acide acétique substitué, de formule générale:

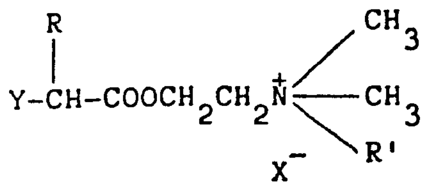

(I)

dans laquelle

R est l'hydrogéne ou un alcoyle inférieur en $C_1$—$C_3$,

R' est un alcoyle linéaire ou ramifié en $C_8$—$C_{16}$,

$X^-$ est $Cl^-$, $Br^-$ or $I^-$,

Y est un radical choisi parmi p-isobutylphényl, 3-benzoyl-phényl, 1-(p-chlorobenzoyl)-2-méthyl-5-méthoxy-3-indolyl, 6-méthoxy-2-naphthyl et 2-fluoro-4-biphényl, caractérisé en ce qu'il comprend les étapes de:

(a) l'estérification du diméthyl-amino-éthanol avec le chlorure d'acide de formule générale:

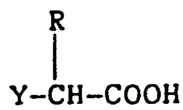

(II)

dans laquelle R et Y sont comme définis ci-dessus et

(b) la réaction de l'ester ainsi obtenu avec un halogénure d'alcoyle de formule générale:

R'X (III)

dans laquelle R' et X sont comme définis ci-dessus.